# FASCICULE DE BREVET EUROPEEN

(11) **EP 3 286 167 B1**
(45) Date de publication et mention de la délivrance du brevet: **24.04.2019**
(21) Numéro de dépôt: 16721196.0
(22) Date de dépôt: 15.04.2016
(51) Int. Cl.: C07C 319/28, C07C 323/58

(54) **PROCEDE DE FABRICATION DE METHIONINE**
VERFAHREN ZUR HERSTELLUNG VON METHIONIN
METHOD FOR MAKING METHIONINE

(30) Priorité: 21.04.2015 FR 1553547
(43) Date de publication de la demande: 28.02.2018
(73) Titulaire: Adisseo France S.A.S., 92160 Antony (FR)
(72) Inventeur: CAPELLE, Nicolas, 69960 Corbas (FR); REY, Patrick, 69003 Lyon (FR)
(74) Mandataire: Agasse, Stéphane
(86) Numéro de dépôt international: PCT/FR2016/050883
(87) Numéro de publication internationale: WO 2016/170252

(56) Documents cités:
- US-A- 2 557 920
- US-A- 6 126 972

## Description

L'invention porte sur une amélioration du procédé de fabrication de la méthionine à partir de l'hydantoïne de la méthionine.

La méthionine est un acide aminé essentiel chez l'homme, elle doit donc être apportée par son alimentation. Mais son marché majeur est celui de la nutrition animale pour laquelle elle est produite en centaines de milliers de tonnes par an. Elle est essentiellement fabriquée par synthèse chimique.

Un des procédés chimiques connus pour l'obtention de la méthionine, voir par exemple US2557920A, comporte une étape de conversion de l'hydantoïne de la méthionine par hydrolyse alcaline, ou saponification, en sel de méthioninate, puis neutralisation de ce dernier en méthionine. L'hydrolyse de l'hydantoïne de la méthionine est effectuée en présence de soude et/ou de carbonate de sodium, en phase aqueuse. Elle se manifeste par une élimination simultanée de dioxyde de carbone et d'ammoniac qui conduit au sel de méthionine, le méthioninate de sodium. Le dioxyde de carbone et l'ammoniac sont éliminés du milieu d'hydrolyse, puis le milieu réactionnel d'hydrolyse contenant le sel de méthioninate est neutralisé par de l'acide sulfurique pour conduire à la méthionine. Celle-ci est ensuite séparée et purifiée par cristallisation.

Les conditions réactionnelles classiques des étapes précitées sont les suivantes :
L'hydrolyse alcaline peut être réalisée en présence d'un excès d'hydroxyde de sodium, de 1,3 à 3,5 éq., de préférence de 1,7 à 2,5 éq., pour des températures comprises entre 150 et 200°C.

Après élimination de l'ammoniac et du dioxyde de carbone du milieu de saponification, la neutralisation est généralement réalisée par addition d'acide sulfurique concentré jusqu'à l'obtention d'un pH compris entre 3 et 6 à des températures comprises entre 70 et 130°C. La méthionine peut alors être purifiée par cristallisation, par la suite, par refroidissement. La méthionine est alors séparée du sulfate de sodium formé, par filtration à 30-50°C.

L'inconvénient prédominant de cette synthèse est la formation d'importants volumes de sulfate de sodium, à l'issue de la neutralisation, qui ne peuvent pas être recyclés dans le procédé de fabrication de la méthionine et qui sont difficilement valorisables.

L'art antérieur apporte quelques réponses à ce problème, avec des procédés de production de solutions aqueuses de méthioninate de sodium qui sont utilisées à la place de la méthionine solide. Selon ces procédés, on réalise une saponification de l'hydantoïne de la méthionine en présence de soude, et une partie du carbonate de sodium formé lors de l'hydrolyse alcaline est séparée et peut être recyclée à l'étape de saponification, permettant d'obtenir une solution aqueuse de méthioninate moins concentrée en carbonate de sodium. Ainsi selon US4391987A, le carbonate de sodium est séparé par précipitation à froid, à une température variant de -10°C à 5°C. Ceci présente le désavantage d'utiliser des fluides frigorifiques et devoir refroidir une solution de saponification se trouvant à plus de 100°C, ce qui entraîne un coût énergétique significativement élevé. US6126972A apporte une amélioration à cette séparation en concentrant les solutions de saponification pour faire précipiter à chaud (100-130°C) le carbonate de sodium et le séparer par filtration à chaud (100-130°C). Le but de cette séparation est d'obtenir du méthioninate de sodium qui peut être granulé par la suite par l'intermédiaire d'un lit fluidisé.

L'invention s'inscrit dans le développement d'un procédé permettant la production de méthionine solide, de haute pureté, qui présente l'avantage d'être beaucoup plus efficace pour la nutrition animale, à poids égal que son sel, le méthioninate de sodium, et à ce jour, il n'est toujours pas remédié à la co-production excessive de sulfate de sodium lors de la neutralisation du méthioninate en méthionine.

L'invention apporte une solution avec un procédé de fabrication de la méthionine comportant une étape supplémentaire permettant de réduire considérablement la quantité de sulfate de sodium formé. Ce procédé peut être mis en oeuvre à l'échelle industrielle via un procédé continu ou discontinu. Les rendements en méthionine sont quasi quantitatifs et la qualité de la méthionine n'est pas affectée par la modification apportée.

Ainsi, le procédé de l'invention comprend l'hydrolyse alcaline de l'hydantoïne de la méthionine en phase aqueuse, l'élimination de NH₃ et CO₂ du milieu d'hydrolyse, et la neutralisation du sel de méthioninate, ledit procédé comportant une étape de concentration du milieu réactionnel d'hydrolyse, après élimination de NH₃ et CO₂, cette concentration permettant de précipiter du Na₂CO₃, ledit Na₂CO₃ étant séparé puis recyclé à l'étape précédente d'hydrolyse alcaline. L'étape d'hydrolyse alcaline du procédé de l'invention est donc opérée en présence de NaOH et de Na₂CO₃, ce qui permet, d'une part, de réduire significativement la quantité de soude nécessaire, cette réduction étant compensée par un apport en carbonate de sodium provenant du procédé lui-même, et d'autre part, de diminuer la quantité de sulfate de sodium formé à l'issue de la neutralisation. Un abaissement de 20 à 50% (m/m) de la quantité de sulfate de sodium par kg de méthionine produite peut être mesuré.

Le milieu d'hydrolyse comprend le méthioninate de sodium et le carbonate de sodium qui présentent des profils de solubilité différents, en fonction de la température et de leurs concentrations respectives. La difficulté réside dans un compromis reproductible entre une cristallisation d'une quantité maximale de carbonate de sodium et celle d'une quantité minimale du méthioninate de sodium qui permettra de déterminer les conditions de neutralisation du méthioninate en méthionine. Les inventeurs ont d'abord observé qu'une étape d'extraction de carbonate de sodium pouvait être insérée dans un procédé industriel de synthèse de la méthionine afin de résoudre le problème de coproduction de sulfate de sodium et ils ont en outre défini des conditions optimales dans lesquelles le compromis ci-dessus était atteint afin de ne pas affecter le rendement de l'étape de neutralisation pour obtenir la méthionine. Elles sont décrites ci-après et peuvent être considérées seules ou en combinaison.

Avantageusement, la concentration, ou enrichissement, du milieu en carbonate de sodium est réalisée par élimination de l'eau du milieu jusqu'à une concentration du sel de méthioninate variant de 20 à 70%, avantageusement de 30 à 50% exprimé en masse de méthioninate de sodium par rapport à la masse du milieu. Toute technique bien connue de l'homme du métier peut être mise en oeuvre pour concentrer le milieu. Une élimination de l'eau par évaporation à une température variant de 90 à 100°C, sous pression atmosphérique, est particulièrement efficace. Elle permet une cristallisation d'une quantité maximale de carbonate de sodium tout en maintenant une teneur maximale de sel de méthioninate en solution dans le milieu pour l'étape suivante de neutralisation. Selon une autre variante, l'élimination de l'eau est effectuée par évaporation sous vide à une température variant de 30 à 90°C, de préférence 40 à 60°C.

Le milieu d'hydrolyse alcaline de l'hydrantoïne peut comprendre l'hydantoïne de méthionine pure, l'hydantoïne non purifiée ou partiellement purifiée depuis son milieu de synthèse. L'hydantoïne de la méthionine peut être obtenue à partir de 2-hydroxy-4-méthylthio-butyronitrile (HMTBN). Dans ce cas, elle n'est de préférence pas isolée du milieu réactionnel et ce dernier est directement soumis aux conditions de saponification. Il est alors préférable que la proportion de l'hydantoïne de la méthionine dans ledit milieu réactionnel soit d'au moins 10%. Avantageusement, une élimination préalable de l'ammoniac et du dioxyde de carbone produits lors de la synthèse de l'hydantoïne de méthionine est réalisée.

De préférence, pour l'hydrolyse alcaline de l'hydantoïne de la méthionine, le rapport molaire de la somme des bases sur la somme des produits soufrés (Na/S) est d'au moins 2, de préférence compris entre 2,5 et 4. Un tel rapport permet d'atteindre des rendements de saponification supérieurs à 98%. Il permet en particulier de limiter la formation des dipeptides de méthionine. La quantité de soufre est, bien entendu, majoritairement apportée par l'hydantoïne de la méthionine du milieu réactionnel où elle est formée à partir de l'HMTBN ; elle provient aussi, en quantités mineures, de l'HMTBN non consommé et de l'hydantoïne de la méthionine présente à l'état d'impuretés dans le carbonate de sodium recyclé et introduit dans le milieu de saponification. Le sodium est apporté par la soude ajoutée en tant que réactif de saponification et par le carbonate de sodium récupéré à l'issue de la concentration du milieu d'hydrolyse et recyclé. De préférence, le rapport molaire NaOH/Na₂CO₃ dans le milieu d'hydrolyse varie de 0,5 à 3, de préférence 1 à 2.

Le carbonate de sodium cristallisé dans le milieu d'hydrolyse est séparé par toute technique bien connue de l'homme du métier, par exemple par filtration. Selon une variante avantageuse du procédé de l'invention, le carbonate de sodium est filtré à une température variant de 70 à 130°C, de préférence de 90 à 110°C. Cette filtration à chaud est effectuée après une concentration suffisante du flux de saponification pour faire précipiter la quantité désirée de carbonate de sodium.

Dans une mise en oeuvre préférée du procédé de l'invention, après séparation, le Na₂CO₃ récupéré est préalablement dissous dans l'eau éliminée lors de la concentration du milieu d'hydrolyse en carbonate de sodium, puis recyclé à l'étape d'hydrolyse de l'hydantoïne de méthionine.

Le flux de méthioninate de sodium peut être ensuite neutralisé, par de l'acide sulfurique comme dans le procédé classique de fabrication de la méthionine, mais aussi par du dioxyde de carbone, sous forme de gaz sous pression, qui permet de neutraliser le milieu sous forme de bicarbonate de sodium et de méthionine, qui précipite du milieu et peut être séparée par filtration. La neutralisation est avantageusement effectuée à une température variant de 10 à 60°C.

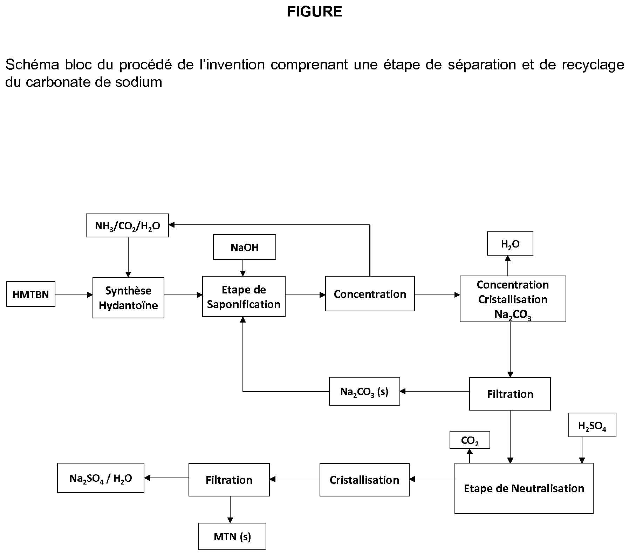

Le procédé de l'invention est illustré dans les exemples suivants, à l'appui de la figure unique suivante.

La figure montre le schéma bloc de synthèse de la méthionine intégrant une étape de concentration du milieu d'hydrolyse en carbonate de sodium, selon l'invention. La méthionine est fabriquée à partir de 2-hydroxy-4-méthylthio-butyronitrile (HMTBN) qui est converti en hydantoïne de méthionine par toute technique connue, telle qu'une hydrolyse ammoniacale, en présence de bicarbonate d'ammonium. L'hydantoïne de méthionine est ensuite saponifiée par de la soude, puis le jus de saponification est neutralisé. Conformément à l'invention, une étape de concentration des jus de saponification est ainsi ajoutée, elle permet de faire précipiter le carbonate de sodium, qui est séparé du méthioninate de sodium par filtration, remis en suspension aqueuse puis recyclé à l'étape de saponification. Par rapport au procédé classique, la co-production de sulfate de sodium est fortement réduite.

### Exemple 1 : Etape de saponification

Dans un autoclave, on charge 92 g de soude à 50% (m/m), 52 g de carbonate de sodium et 520 g de flux de synthèse de l'hydantoïne de méthionine, contenant 139 g d'hydantoïne et de dérivés (acide hydantoïque, méthioninamide, uréido-butyramide). Le milieu est chauffé à 180°C pendant 30 min, la pression autogène s'élève à 10 bars. Après refroidissement, le dosage du milieu réactionnel par chromatographie en phase liquide permet de calculer un rendement en méthionine supérieur à 98%. Avant d'être engagé dans l'étape de concentration aux fins de cristallisation du carbonate de sodium, le flux de saponification est pré-concentré par stripping à l'azote.

### Exemple 2 : Etape de séparation du carbonate de sodium

1100 g d'un flux de saponification issu de la concentration préalable ci-dessus et contenant 150 g de méthioninate de sodium et 110 g de carbonate de sodium est concentré à 90-110°C par évaporation. 600 g d'eau contenant du carbonate d'ammonium sont ainsi éliminés. Le milieu obtenu, dans lequel une partie du carbonate de sodium a précipité, est filtré à 90°C sous pression d'azote (150 mbar). 165 g d'un solide, contenant du carbonate de sodium et du méthioninate de sodium, est séparé, qui peut être recyclé à l'étape de saponification. 340 g de jus mère contenant 125 g de méthioninate de sodium sont récupérés pour l'étape suivante de neutralisation.

### Exemple 3 : Etape de neutralisation/Cristallisation de la Méthionine

Dans un réacteur, on charge 740 g de jus mères de filtration du carbonate de sodium obtenu à l'exemple 2, contenant 190 g de méthioninate de sodium et 50 g de carbonate de sodium n'ayant pas cristallisé à l'étape précédente. Le milieu obtenu est neutralisé à 90-100°C jusqu'à un pH∼4,5 par l'ajout de 135 g d'acide sulfurique à 92,5%. La méthionine est cristallisée dans le flux par refroidissement à 40°C. Elle est séparée par filtration sous pression d'azote et après lavage avec 100 g d'eau, on obtient 200 g de jus contenant 2,2% de méthionine. Après séchage à 100°C, 170 g de gâteau sec de méthionine sont obtenus à 95% de pureté et contenant 4% de sulfate de sodium résiduel.

## Revendications

1. Procédé continu de fabrication de la méthionine par hydrolyse alcaline de l'hydantoïne de la méthionine en phase aqueuse, élimination de NH₃ et CO₂ du milieu d'hydrolyse, et neutralisation du sel de méthioninate obtenu, **caractérisé en ce que**, après élimination de NH₃ et CO₂, le milieu réactionnel d'hydrolyse est concentré pour précipiter du Na₂CO₃, ledit Na₂CO₃ étant séparé puis recyclé pour l'hydrolyse alcaline, celle-ci étant réalisée en présence de NaOH et de Na₂CO₃.

2. Procédé selon la revendication 1, **caractérisé en ce que** le milieu réactionnel d'hydrolyse contient du sel de méthioninate et **en ce que** la concentration est réalisée par élimination de l'eau du milieu jusqu'à une concentration du sel de méthioninate variant de 20 à 70%, en masse de méthioninate de sodium par rapport à la masse du milieu, de préférence de 30 à 50%.

3. Procédé selon la revendication 2, **caractérisé en ce que** l'élimination de l'eau est effectuée par évaporation à une température variant de 90 à 110°C, sous pression atmosphérique.

4. Procédé selon la revendication 2, **caractérisé en ce que** l'élimination de l'eau est effectuée par évaporation sous vide à une température variant de 30 à 90°C, de préférence 40 à 60°C.

5. Procédé selon l'une quelconque des revendications 1 à 4, **caractérisé en ce que** le Na₂CO₃ est séparé par filtration à une température variant de 70 à 130°C.

6. Procédé selon l'une quelconque des revendications 1 à 5, **caractérisé en ce qu'**après séparation, le Na₂CO₃ est dissous dans l'eau évaporée puis recyclé à l'hydrolyse de l'hydantoïne de la méthionine.

7. Procédé selon l'une quelconque des revendications 1 à 6, **caractérisé en ce que** le rapport molaire Na/S pour l'hydrolyse alcaline de l'hydantoïne de la méthionine est d'au moins 2,0, de préférence compris entre 2,5 et 4.

8. Procédé selon la revendication 7, **caractérisé en ce que** le rapport molaire de NaOH/Na₂CO₃ varie de 1 à 3.

9. Procédé selon l'une quelconque des revendications 1 à 8, **caractérisé en ce que** le milieu d'hydrolyse est neutralisé par de l'acide sulfurique.

10. Procédé selon l'une quelconque des revendication 1 à 8, **caractérisé en ce que** le milieu d'hydrolyse est neutralisé par du dioxyde de carbone.

11. Procédé selon la revendication 9 ou 10, **caractérisé en ce que** le milieu d'hydrolyse est neutralisé à une température variant de 10 à 60°C.

12. Procédé selon l'une quelconque des revendications 1 à 11, **caractérisé en ce que** l'hydantoïne de la méthionine est obtenue à partir de 2-hydroxy-4-méthylthio-butyronitrile (HMTBN) et n'est pas isolée du milieu réactionnel.

13. Procédé selon la revendication 12, **caractérisé en ce que** la proportion de l'hydantoïne de la méthionine dans ledit milieu réactionnel est d'au moins 10%.

## Patentansprüche

1. Kontinuierliches Verfahren zur Herstellung des Methionins durch alkalische Hydrolyse des Hydantoins des Methionins in wässriger Phase, Eliminieren von NH₃ und CO₂ des Hydrolysemediums und Neutralisieren des erhaltenen Methioninatsalzes, **dadurch gekennzeichnet, dass**, nach dem Eliminieren von NH₃ und CO₂, das Reaktionsmedium der Hydrolyse konzentriert wird, um Na₂CO₃ auszufällen, wobei das Na₂CO₃ für die alkalische Hydrolyse getrennt und dann recycelt wird, wobei dieselbe in Anwesenheit von NaOH und von Na₂CO₃ durchgeführt wird.

2. Verfahren nach Anspruch 1, **dadurch gekennzeichnet, dass** das Reaktionsmedium der Hydrolyse Methioninatsalz enthält, und dadurch, dass die Konzentration durch Eliminieren des Wassers des Mediums bis zu einer Konzentration des Methioninatsalzes durchgeführt wird, die von 20 bis 70 Massenprozent Natriummethioninat mit Bezug auf die Masse des Mediums variiert, vorzugsweise von 30 bis 50 %.

3. Verfahren nach Anspruch 2, **dadurch gekennzeichnet, dass** das Eliminieren des Wassers durch Verdampfen bei einer Temperatur, die von 90 bis 110 °C variiert, unter atmosphärischem Druck durchgeführt wird.

4. Verfahren nach Anspruch 2, **dadurch gekennzeichnet, dass** das Eliminieren des Wassers durch Verdampfen unter Vakuum bei einer Temperatur durchgeführt wird, die von 30 bis 90 °C variiert, vorzugsweise von 40 bis 60 °C.

5. Verfahren nach einem der Ansprüche 1 bis 4, **dadurch gekennzeichnet, dass** das Na₂CO₃ durch Filtrieren bei einer Temperatur durchgeführt wird, die von 70 bis 130 °C variiert.

6. Verfahren nach einem der Ansprüche 1 bis 5, **dadurch gekennzeichnet, dass** nach dem Trennen das Na₂CO₃ im verdampften Wasser gelöst, dann mit der Hydrolyse des Hydantoins des Methionins recycelt wird.

7. Verfahren nach einem der Ansprüche 1 bis 6, **dadurch gekennzeichnet, dass** das Molverhältnis Na/S für die alkalische Hydrolyse des Hydantoins des Methionins mindestens 2,0 beträgt, vorzugsweise im Bereich zwischen 2,5 und 4 liegt.

8. Verfahren nach Anspruch 7, **dadurch gekennzeichnet, dass** das Molverhältnis NaOH/Na₂CO₃ von 1 bis 3 variiert.

9. Verfahren nach einem der Ansprüche 1 bis 8, **dadurch gekennzeichnet, dass** das Hydrolysemedium durch Schwefelsäure neutralisiert wird.

10. Verfahren nach einem der Ansprüche 1 bis 8, **dadurch gekennzeichnet, dass** das Hydrolysemedium durch Kohlendioxid neutralisiert wird.

11. Verfahren nach Anspruch 9 oder 10, **dadurch gekennzeichnet, dass** das Hydrolysemedium bei einer Temperatur neutralisiert wird, die von 10 bis 60 °C variiert.

12. Verfahren nach einem der Ansprüche 1 bis 11, **dadurch gekennzeichnet, dass** das Hydantoin des Methionins ausgehend von 2-Hydroxy-4-methylthio-butyronitril (HMTBN) erhalten wird und nicht vom Reaktionsmedium isoliert wird.

13. Verfahren nach Anspruch 12, **dadurch gekennzeichnet, dass** die Proportion des Hydantoins des Methionins in dem Reaktionsmedium mindestens 10 % beträgt.

## Claims

1. A continuous method for producing methionine by alkaline hydrolysis of methionine hydantoin in an aqueous phase, elimination of NH₃ and CO₂ from the hydrolysis medium, and neutralization of the obtained methioninate salt, **characterized in that**, after elimination of NH₃ and CO₂, the hydrolysis reaction medium is concentrated so as to precipitate Na₂CO₃, said Na₂CO₃ being separated and then recycled for alkaline hydrolysis, which is carried out in the presence of NaOH and Na₂CO₃.

2. The production method according to claim 1, **characterized in that** the hydrolysis reaction medium contains methioninate salt and **in that** the concentration is carried out by eliminating water from the medium to a concentration of the methioninate salt varying from 20 to 70%, in sodium methioninate mass with respect to the medium mass, preferably from 30 to 50%.

3. The production method according to claim 2, **characterized in that** the elimination of water is carried out by evaporation at a temperature varying from 90 to 110°C, under atmospheric pressure.

4. The production method according to claim 2, **characterized in that** the elimination of water is performed by vacuum evaporation at a temperature varying from 30 to 90°C, preferably from 40 to 60°C.

5. The production method according to any one of claims 1 to 4, **characterized in that** Na₂CO₃ is separated by filtration at a temperature varying from 70 to 130°C.

6. The production method according to any one of claims 1 to 5, **characterized in that** after separation, Na₂CO₃ is dissolved in the evaporated water and then recycled in the hydrolysis of methionine hydantoin.

7. The production method according to any one of claims 1 to 6, **characterized in that** the Na / S molar ratio for the alkaline hydrolysis of methionine hydantoin is at least 2.0, preferably comprised between 2.5 and 4.

8. The production method according to claim 7, **characterized in that** the NaOH / Na₂CO₃ molar ratio varies from 1 to 3.

9. The production method according to any one of claims 1 to 8, **characterized in that** the hydrolysis medium is neutralized with sulfuric acid.

10. The production method according to any one of claims 1 to 8, **characterized in that** the hydrolysis medium is neutralized with carbon dioxide.

11. The production method according to claim 9 or 10, **characterized in that** the hydrolysis medium is neutralized at a temperature varying from 10 to 60°C.

12. The production method according to any one of claims 1 to 11, **characterized in that** methionine hydantoin is obtained from 2-hydroxy-4-methylthio-butyronitrile (HMTBN) and is not isolated from the reaction medium.

13. The production method according to claim 12, **characterized in that** the proportion of methionine hydantoin in said reaction medium is at least 10%.
